# EUROPEAN PATENT SPECIFICATION

(11) **EP 0 804 072 B1**
(45) Date of publication and mention of the grant of the patent: **14.05.2003**
(21) Application number: 95926162.9
(22) Date of filing: 30.06.1995
(51) Int. Cl.: A01N 1/02, A01N 63/00, A01N 65/00, C12N 5/00, C12N 5/02, C12M 3/00, C12M 3/02, C12M 1/24, A61K 35/14

(54) **Method of cultivating macrophages**
Verfahren zur Kultivierung von Makrophagen
Procédé de mise en culture de macrophages

(30) Priority: 03.07.1994 IL 11019594
(43) Date of publication of application: 05.11.1997
(73) Proprietor: Crotty, Daphna, Bethesda MD 20814 (US); Danon, David, 55900 Ganei Tiqva (IL)
(72) Inventor: DANON, David, 55900 Ganei Tiqva (IL)
(74) Representative: Ebner von Eschenbach, Jennifer
(86) International application number: US9508351
(87) International publication number: WO96001045

(56) References cited:
- EP-A- 0 402 698
- EP-A- 0 479 309
- EP-A- 0 573 405
- EP-A- 0 600 804
- WO-A-92/15274
- WO-A-94/26875
- WO-A-96/22781
- CH-A- 570 457
- US-A- 4 004 587
- US-A- 4 342 828
- US-A- 4 361 148
- US-A- 4 757 017
- US-A- 4 829 002
- US-A- 4 919 823
- US-A- 4 937 194
- US-A- 4 938 758
- US-A- 4 946 434
- US-A- 4 997 762
- US-A- 5 070 012
- US-A- 5 098 371
- US-A- 5 141 486
- US-A- 5 192 553
- US-A- 5 236 716
- PATENT ABSTRACTS OF JAPAN vol. 017, no. 001 (C-1014), 5 January 1993 & JP 04 236959 A (TERUMO CORP), 25 August 1992
- DATABASE WPI Section Ch, Week 8828 Derwent Publications Ltd., London, GB; Class B04, AN 88-193673 XP002096192 & JP 63 130600 A (AGENCY OF IND SCI & TECHNOLOGY), 2 June 1988
- DATABASE WPI Section Ch, Week 9326 Derwent Publications Ltd., London, GB; Class B04, AN 93-208286 XP002096193 & JP 05 130863 A (JAPAN TOBACCO INC) , 28 May 1993
- CHOKRI M. ET AL.: "PRODUCTION OF HUMAN MACROPHAGES WITH POTENT ATITUMOR PROPERTIES (MAK) BY CULTURE OF MONOCYTES IN THE PRESENCE OF GM-CSF AND 1,25- DIHYDROXY VITAMIN D3" ANTICANCER RESEARCH, vol. 12, no. 6b, November 1992, pages 2257-2260, XP002096191

## Description

The present invention relates to a method and system for cultivating cells. The method and system are particularly useful for growing macrophages from human blood, and are therefore described below with respect to this application.

Macrophages, namely phagocytic cells phagocytize and digest old and deteriorated red blood cells, bacteria and other microscopic particles. They have been found to play an important role in wound repair. They produce substances that stimulate proliferation of fibroblasts, the synthesis of collagen by fibroblasts, and other elements that are necessary for wound healing. For example, it was found that wound repair could be accelerated in old mice by application of macrophages derived from peritoneal fluids of young mice (D.Danon, M.A.Kowatch and G.S.Roth, Proc. Natl. Acad. Sci. USA, Vol.86, pp.2018-2020, March 1989).

US 5,236,716 teaches a platelet concentrate containing less than 10⁶ white blood cells and prepared from a unit of whole blood sample in a closed multiple blood bag system less than about eight hours after the whole blood is removed from a human, the concentrate being contained in a platelet storage bag made from polyvinyl chloride plasticized with one from the group of plasticizers consisting of trioctyltrimellitate and ethylene vinyl acetate.

US 5,070,012 teaches a method for studying a regulatory element of a transcriptional initiation regulatory system in a viable mammalian cell, wherein the cell comprises a non-wild type expression construct comprising a transcriptional initiation regulatory region comprising a regulatory sequence of interest and a structural gene encoding β-galactosidase, wherein the β-galactosidase employs as a substrate a β-galactosidyl ether of a fluorescent phenolic compound that produces a fluorescent product upon hydrolysis of said ether, the method comprising: substantially irreversibly introducing the substrate into such a cell in a hypotonic solution at a temperature in the range of about 25°-40°C; incubating the cell at a temperature below about the membrane freezing point; and detecting the fluorescent product in the cell as a measure of the activity of said regulatory element.

Chockri et al., Anticancer Research 12:2257-2260 (1992) disclose a process for preparing macrophages by culturing monocytes in a culture medium containing 1,25-dihydroxy D3 vitamin and GM-CSF. The macrophages so obtained are alleged to have either (a) cytotoxic activity without inteferon-gamma (IFN-γ) increased by 20-30% with respect to standard macrophages, (b) cytotoxic activity with IFN-γ increased by about 20 to 40% with respect to standard macrophages, or (c) extension of the deactivation of cytotoxic activity in response to activation of IFN-γ in a ration such that after 60h activation with IFN-γ, the cytotoxic activity is higher than or equal to 30% compared to the maximum cytotoxic activity presented by the macrophages due to IFN-γ activation, with the cytotoxic activity being measured as the percentage of inhibition of 30H thymidine incorporation by target tumoral cells (U 937 cells).

Derwent World Patents Index abstract accession number 88-193673 states that JP 63-130600 discloses a macrophage-like cell derived from a human cell is cultured with a macrophage activating substance. The macrophage-like cell is reportedly prepared from a culture of peripheral blood cell monocyte, tissue macrophage, macrophage precursor cell (e.g. HL-60 cell, THP-1 cell of Mono-1-207 cell) in vitro with differentiation derivation agents (e.g. phorbol ester, diterpenoids, etc.). The macrophage activating substance is stated to be a vitamin A derivative (e.g. vitamin A acid, vitamin A alcohol, vitamin A acetate, etc.); DMSO, hydrocortisone, lipopolysaccharide, etc.

The present methods of preparing macrophages out of blood monocytes known in the prior art are complicated, expensive, time-consuming and difficult to apply routinely, because they require considerable specialized labour, expensive disposable materials, and specialized laboratory facilities. Moreover, these techniques involve a significant risk of contamination during preparation and therefore require regular testing to assure the absence of bacterial infection in the resulting suspension of macrophages.

An object of the present invention is to provide a method and system for growing cells, particularly, macrophages, which have advantages in the above respects. More particularly, an object of the invention is to provide a method and system for growing cells, particularly, macrophages, which do not require specialized labour, expensive disposable materials, or a specialized laboratory, and which, in one aspect, reduce considerably the risk of infection.

According to the present invention, there is provided a method of cultivating macrophages, characterized by the steps of separating from an initial quantity of blood, a white blood cell fraction which includes a mixture of white blood cells and red blood cells having a higher concentration of white blood cells than in the initial quantity of blood; subjecting said white blood cell fraction to an osmotic shock which is more destructive of red blood cells than white blood cells; re-establishing iso-tonicity in said white blood cell fraction; adding said white blood cell fraction to a culture medium in a container to obtain a suspension of the white blood cells; and incubating said culture medium.

In one aspect the macrophages are cultivated by subjecting the white blood cells to at least one sterile reagent and cultivating the cells in a sterile culture medium, comprising: placing the cells in suspension, the at least one reagent, and the culture medium in three separate sterile containers; connecting together the containers, and hermetically sealing their contents from the atmosphere, by sterile tubings having fluid flow control devices which may be opened and closed; and opening and closing the fluid flow control devices as required in order to transfer the contents of one container to another, while isolating the container contents from the external environment.

The invention is particularly useful for cultivating macrophages from blood.

Since the complete process can thus be carried out in a completely controlled atmosphere hermetically sealed from the outside atmosphere, there is little risk of infection, and therefore no need for special sterile hoods, laminary flows, and sterile disposable tools. Testing to assure the absence of bacterial infection is not crucial. In fact, not one case of infection was found in 150 preparations of macrophages in accordance with the method of the present invention where every second or third preparation was tested.

It has been found that subjecting the white blood cell fraction to osmotic shock and then re-establishing iso-tonicity therein destroys substantially all the red blood cells and much fewer white blood cells, such that the result is a suspension having a high concentration of white blood cells suitable for cultivation in a container with a culture medium. It has also been found that this osmotic shock treatment of the monocytes in the white blood cells fraction enhances their differentiation into macrophages, as evidenced morphologically.

Preferably, the white cell fraction is subjected to osmotic shock by mixing it with a 15-fold volume of distilled water for a period of 30-90 seconds; a period of 60 seconds has been found particularly effective. The iso-tonicity is also preferably re-established by adding to the white blood cell fraction a hypertonic solution of one tenth of the volume of the distilled water used and ten times the concentration of iso-tonic solution, preferably a 9% solution of NaCl.

According to a further important feature of the invention, a serum prepared from the initial quantity of blood is added to the separated white blood cells in the culture medium. This serum is prepared by separating a desired volume of a plasma from the initial quantity of blood, adding a coagulating agent to the plasma to produce the serum, and then separating the serum from the coagulated plasma. Prior procedures for preparing macrophages introduced fetal calf serum or pooled human serum but there is a danger that fetal calf serum may cause creation of antibodies against this serum in the treated host, and pooled human serum increases the probability of adding the danger of viral infection. These dangers are avoided by using serum prepared from the same blood from which the white blood cells were separated.

According to a still further aspect of the present invention, there is provided apparatus for cultivating cells present in suspension by subjecting the cells to at least one sterile reagent and cultivating the cell in a sterile culture medium, comprising: a first container for receiving the cells in suspension; a second container for receiving the at least one reagent; and a third container for receiving the culture medium; the containers being connected together and hermetically sealed from the atmosphere by sterile tubings having fluid flow control devices which may be opened and closes at required in order to transfer the contents of one container to another, while isolating the container contents from the external environment.

While it was previously known, as indicated above, that wound repair could be accelerated in old mice by application to their wounds of macrophages derived from peritoneal fluids of young mice, insofar as the applicant is aware it was not known to use macrophages derived from blood for the therapeutic treatment of a living body, and particularly for wound healing.

According to a still further aspect of the present invention, therefore, there is provided a method for the therapeutic treatment of a living body by applying thereto macrophages cultivated from blood.

Further features and advantages of the invention will be apparent from the description below of a specific example of a method of growing macrophages in accordance with the present invention, using a system as illustrsted in the single accompanying drawing figure.

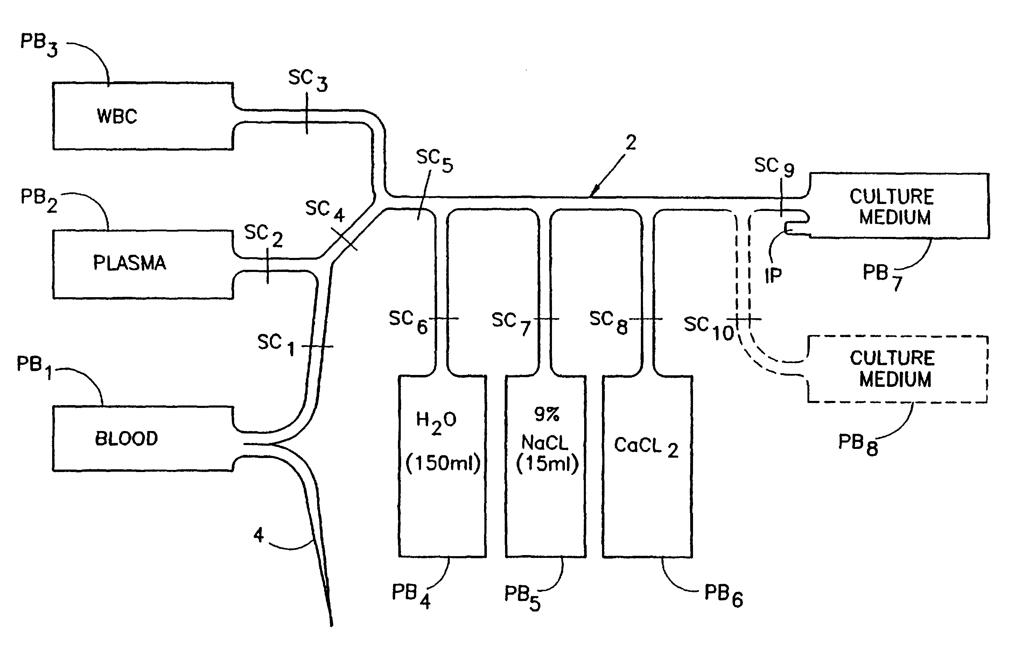

The following example describes a procedure for growing macrophages out of white blood cells contained in a quantity of human blood as obtained from the blood bank. The described procedure is carried out in a totally enclosed system without exposing the cells or the various media involved in the procedure to the external environment. Before the procedure is started, the system illustrated in the drawing is setup by providing the following containers in the form of flexible plastic bags PB₁-PB₈: plastic bag PB₁, for containing the initial quantity of blood to be processed; plastic bags PB₂ and PB₃, for receiving a plasma fraction and a white blood cell fraction, respectively, separated from the initial quantity of blood in plastic bag PB₁ during the procedure; plastic bags PB₄, PB₅ and PB₆ for receiving distilled water (150 ml), a 9% solution of NaCl (15 ml), and CaCl₂, respectively, used as reagents during the procedure; and plastic bag PB₇, to contain a culture medium to be used in the procedure. If desired, an eighth plastic bag PB₈ may be included also to contain a culture medium. Preferably, plastic bags PB₇ and/or PB₈ contains an injection port IP (shown only in plastic bag PB₇) for injecting one or more additives to the culture medium within that bag, as will be described more particularly below.

All the plastic bags PB₁-PB₈ are interconnected together by tubings generally designated 2, which hermetically seal the contents of each bag from the atomosphere. The illustrated tubings 2 include a plurality of manual valves, in the form of slide clamps SC₁-SC₁₀, which may be manually closed in order to seal the contents of each bag from the others, or manually opened in order to permit transfer of the contents of one bag to another during the various stages of the procedure. It will thus be seen that the illustrated system of plastic bags PB₁-PB₈, interconnected together by tubings 2 having the slide clamps SC₁-SC₁₀, isolate all the container contents from the external environment while permitting transfer of the contents of one container to another during the various stages of the procedure.

A quantity of human blood is first collected into bag PB₁ (containing an anti-coagulant) by venipuncture in the blood bank using a sterile needle 4. Bags PB₂ and PB₃ are initially empty. The three bags PB₁, PB₂ and PB₃ are introduced into one container of a centrifuge, while the remaining bags PB₄-PB₈ are introduced into the opposite container of the centrifuge, with all the bags being connected together by tubing 2 and all the slide clamps CC₁-CC₉ closed. The two centrifuge containers are equilibrated.

The bags are centrifuged for five minutes at 2,500 rpm (1,000g). This causes the blood within plastic bag PB₁ to separate into a plasma fraction, a white blood cell (buffy coat) fraction, and a red blood cell fraction. The plasma fraction is transferred into bag PB₂ after opening slide clamps SC₁ and SC₂ and compressing bag PB₁. Then the white blood cell fraction is transferred to bag PB₃ after opening slide clamps SC₁, SC₄, and SC₃.

At this stage, a part of the plasma fraction is returned to the red blood cell fraction in plastic bag PB₁ after opening slide clamps SC₃, SC₄ and SC₁. Bag PB₁ may then be disconnected near slide clamp SC₁ (after sealing the plastic tubing) and returned to the blood bank for use.

The white blood cell fraction now in bag PB₃ is a mixture of white blood cells with red blood cells, but having a higher concentration of white blood cells than in the initial quantity of blood.

The white blood cell fraction now in bag PB₃ is subjected to an osmotic shock which is more destructive (shock lysis) of the red blood cells than the white blood cells in that bag. This is done by transferring the distilled water (150 ml) in bag PB₄ to bag PB₃, by opening slide clamps SC₆, SC₅ and SC₃. This should be executed as quickly as possible, by placing bag PB₃ flat on a table, with one hand pressing bag PB₄ to discharge the water from that bag into bag PB₃, and using the other hand for mixing the water with the white blood cell fraction in bag PB₃. This mixing is done for a period of between 30 to 90 seconds, best results having been obtained when it is done for 35 seconds. As indicated above, this subjects the white blood cell fraction within bag PB₃ to osmotic shock, destroying most of the red blood cells by shock lysis, thereby substantially increasing the concentration of the white blood cells within that bag.

At this point, i.e., after the distilled water has been mixed with the white blood cell fraction in bag PB₃ for exactly 35 seconds, the 9% NaCl solution in bag PB₅ is introduced into plastic PB₃, by opening slide clamps SC₇, SC₅ and SC₃, and closing slide clamp SC₆ and squeezing bag PB₅ to discharge its contents into bag PB₃. The NaCl solution thus introduced into bag PB₃, being a hypertonic solution, re-establishes iso-tonicity in the white blood cell fraction in bag PB₃.

At this time, the 12 ml of 20mM CaCl₂ solution in bag PB₆ is transferred to the plasma fraction in bag PB₂, by opening slide clamps SC₈, SC₅, SC₄ and SC₂; all the other clamps are closed. The CaCl₂, an agent enabling coagulation, starts the coagulation of the plasma. The plasma fraction in PB₂ may then be placed into a deep freezer, while the rest of the bags hang outside, for ten minutes and then removed. This procedure causes the permeation of the blood platelets with respect to the membranes, and the release of the platelet-derived growth factors. The plasma bag is now placed in a 37°C water bath for 30 minutes to complete the coagulation. If the coagulation is not complete by this time, which would be apparent from the appearance of the contents of the bag, the bag is returned to the 37°C water bath until coagulation is completed.

Slide clamps SC₇ and SC₈ are then closed, and bags PB₅ and PB₆ may then be removed after sealing the plastic tubing near the clamps.

The bag system is then centrifuged at 1,500 rpm (580g) for five minutes. This sediments the coagulated part of the plasma in bag PB₂, leaving the serum as supernatant. It also sediments the white blood cells in bag PB₃. The supernatant liquid in the latter bag is transferred to bag PB₄, by opening slide clamps SC₃, SC₅ and SC₆. Bag PB₄, which originally contained the distilled water, now serves as a sink for receiving the shock lysed cells and the hemolysate in the liquid transferred from bag PB₃, whereas the white blood cells and the few red blood cells that resisted the osmotic shock remain in bag PB₃.

The culture medium in bag PB₇ is now transferred to bag PB₃, by opening slide clamps SC₉, SC₅ and SC₃ to resuspend the white blood cells in bag PB₃. Cell aggregates are dispersed by manipulating bag PB₃, and the suspension is then transferred back to the culture bag PB₇.

The serum in bag PB₂ separated by the centrifugation is introduced into the liquid culture medium in bag PB₇ in an amount constituting 10-100% of the volume of the culture medium. Preferably, the culture medium is RPMI (Rosewell Park Memorial Institute). If e.g., 60 ml of RPMI is in the culture bag PB₇, approximately 6 ml of serum is added to that bag from the plasma bag PB₂. This is done by opening slide clamps SC₂, SC₄, SC₅ and SC₉. At this stage bags, PB₂ and PB₃ can be disconnected by sealing the plastic tubing near slide clamp SC₅.

The injection port IP of culture medium bag PB₇ is then cleaned with ethanol, and an antibiotic mixture of penicillin plus streptomycin including buffer (hepes), is injected by means of a 5 ml syringe with an 18G needle via the injection port IP. The syringe is removed, but the needle is retained in the injection port IP and is used for injecting sterilized air into bag PB₇ by means of an inflating pump that injects the air through an 0.2 micron pore filter, until the bag reaches a thickness of about 3 cm to 4 cm.

The foregoing procedure up to this point usually takes about three hours. The culture bag is then introduced into a 37°C incubator and retained there for at least ten hours, preferably about 20 hours.

After incubation, the supernatant of the culture bag PB₇ is transferred into the empty plastic bag PB₄, serving as a sink. The culture bag PB₇ now contains substantially only the adherent cells. These are the white blood cells or monocytes, that have already acquired morphological characteristics of macrophages, e.g., spindle-shape cells, triangular cells and pseudopods protruding from cells.

About 1O ml of fresh culture medium at 37°C is then introduced from culture bag PB₈ into culture bag PB₇, and the adherent cells in the latter bag are gently rinsed by tilting the bag several times to detach cells that are sedimented but not adherent. This rinsing liquid is then transferred into the "sink" bag PB₄. The rinsing procedure is repeated.

The number of macrophages that are adherent to the inner surface of the plastic bag PB₇ are estimated under an inverted microscope (objective 20, occular 15). At this magnification, the number of macrophages in the bag is equivalent to about 40,000 times the number of macrophages observed within the microscope field (i.e., there are about 40,000 microscope fields on the internal surface of the bag PB₇ in this particular case). Thus, if the field contains 400 macrophages for example, it can be estimated that there would be about sixteen million macrophages within the bag. The rinsing fluid is now transferred to bag PB₄.

If it is desired to have about two million macrophages per ml, about 8 ml of RPMI would be transferred to bag PB₇ from bag PB₈.

At this stage, the culture bag PB₇ is applied to a metallic cold plate at a temperature of about 4°C. This causes the adherent macrophages to detach from the inner surface of bag PB₇. In order to accelerate the detachment of the macrophages, a glass plate is applied over the bag to sandwich the bag between the glass plate and the cold plate while the two plates, and the bag in between, are tilted back and forth several times to produce a mechanical agitation of the liquid within the bag. This procedure takes about 30 minutes.

The cells can now be removed from the bag by using a disposable sterile syringe and needle via the injection port IP. A few drops may be used for counting the macrophages, and according to the results, the macrophages may be concentrated if desired by centrifugation in a sterile, disposable test tube.

While the above procedure describes one example of a method for making macrophages from human blood, it will be appreciated that many variations may be made. For example, hypertonic solutions other than NaCl, coagulating agents other than CaCl₂, and liquid culture media other than RPMI, may be used. Also, other techniques could be used for the initial separation of the white blood cells, e.g., by filtration or by leucophoresis. Further, it was found that if during the incubation period (at least ten hours, preferably 20-24 hours) at 37°C, if the temperature is raised to 41°C for at least one hour (preferably 1.5 hours) during the early part of the incubation period, a "heat shock" was produced which accelerated the differentiation of the monocytes into macrophages.

Another variation would to be include a mixture of 5% CO₂ in the sterilized air introduced into the culture bag, instead of simple air. Also, the bag system may be made up of bag sub-systems connected together as necessary by a Sterile Connection Device. For example, the blood collection system PB₁-PB₃ may be connected to the macrophage processing system of bags PB₄-PB₈ after separation of the while cells in bag PB₃ and disconnection of red cell bag PB₁.

Further, while the invention has been found particularly useful for making macrophages, it will be appreciated that the technique and system using containers, tubings and slide clamps which permit the contents of the various containers to be transferred as desired in a closed hermetically-sealed system, may be advantageously used for cultivating other types of cells without danger of infection from the external atmosphere. The tubings used can -be connected together by conventional connectors, such as rosettes or other multiple-port connectors.

Still further, certain aspects of the invention can be used independently of other aspects. For example, the method of growing macrophages by the above-described, self-serum and closed-system technique could use other techniques than osmotic shock (e.g., hypertonic exposure, heavy metals, etc.) for inducing differentiation of the monocytes into macrophages. The invention could also be used for growing other types of cells than macrophages.

## Claims

1. A method of cultivating macrophages, **characterized by** the steps of separating from an initial quantity of blood, a white blood cell fraction which includes a mixture of white blood cells and red blood cells having a higher concentration of white blood cells than in the initial quantity of blood; subjecting said white blood cell fraction to an osmotic shock which is more destructive of red blood cells than white blood cells; re-establishing iso-tonicity in said white blood cell fraction; adding said white blood cell fraction to a culture medium in a container to obtain a suspension of the white blood cells; and incubating said culture medium.

2. The method according to claim 1, **characterized in that** serum prepared from said initial quantity of blood is added to said white blood cell fraction after iso-tonicity has been re-established and before the white blood cell fraction has been incubated in a culture medium.

3. The method according to claim 2, **characterized in that** said serum is prepared by separating from said initial quantity of blood also a plasma fraction, adding a coagulating agent to said plasma fraction to produce said serum, and separating said serum from the coagulated plasma.

4. The method according to any one of claims 1-3, **characterized in that** the initial quantity of blood, each fraction separated therefrom, and each reagent used in the method, is contained in a separate container, which containers are all connected together and hermetically sealed from the atmosphere by tubings having clamps which may be manually opened and closed in order to permit transfer of the contents of one container to another to thereby isolate all the contents of the containers from the external environment.

5. The method according to any one of claims 1-3, **characterized by** subjecting said white blood cells to at least one sterile reagent and cultivating said white blood cells in a sterile culture medium.

6. The method according to claim 5, **characterized by** the steps of placing said white blood cells, said at least one reagent, and said culture medium in three separate sterile containers; connecting together said containers, and hermetically sealing their contents from the atmosphere, by sterile tubing having fluid flow control devices which may be opened and closed; and opening and closing said fluid flow control devices as required in order to transfer the contents of one container to another, while isolating the container contents from the external environment.

7. The method according to any one of claims 1-6, **characterized by** subjecting said white blood cell fraction to the osmotic shock by mixing the white blood cell fraction with a volume of distilled water for a period of between 30 to 90 seconds, wherein the volume of distilled water is 15 times larger than that of the white blood cell fraction.

8. The method according to claim 7 , **characterized in that** the iso-tonicity of the white blood cell fraction is re-established by adding to the white blood cell fraction a hypertonic solution of one tenth of the volume of the distilled water and ten times the concentration of isotonic solution.

9. The method according to claim 8, **characterized in that** the hypertonic solution comprises a 9% solution of NaCl.

## Patentansprüche

1. Verfahren zum Kultivieren von Makrophagen, **gekennzeichnet durch** die Schritte des Abtrennens einer Leukozytenfraktion von einer Anfangsmenge Blut, wobei die Leukozytenfraktion eine Mischung von Leukozyten und Erythrozyten mit einer höheren Konzentration von Leukozyten enthält als die Anfangsmenge Blut; die Leukozytenfraktion einem osmotischen Schock aussetzen, der für die Erythrozyten in höherem Maße zerstörend ist als für die Leukozyten; Wiederherstellen der Isotonie in der Leukozytenfraktion; Zusetzen der Leukozytenfraktion zu einem Kulturmedium in einem Behälter, um eine Suspension von Leukozyten zu erhalten; sowie Inkubieren des Kulturmediums.

2. Verfahren nach Anspruch 1, **dadurch gekennzeichnet, dass** das aus der Anfangsmenge Blut hergestellts Serum der Leukozytenfraktion zugegeben wird, nachdem die Isotonie wiederhergestellt worden ist und bevor die Leukozytenfraktion in einem Kulturmedium inkubiert worden ist.

3. Verfahren nach Anspruch 2, **dadurch gekennzeichnet, dass** das Serum hergestellt wird, indem von der Anfangsmenge Blut außerdem eine Plasmafraktion abgetrennt wird; Zusetzen eines Gerinnungsmittels zu der Plasmafraktion, um das Serum zu erzeugen und Abtrennen des Serums von dem geronnenen Plasma.

4. Verfahren nach einem der Ansprüche 1 bis 3, **dadurch gekennzeichnet, dass** die Anfangsmenge Blut, jede davon abgetrennte Fraktion und jedes in dem Verfahren zur Anwendung gelangte Reagens in einem separaten Behälter enthalten sind, wobei die Behälter alle untereinander verbunden und gegenüber der Atmosphäre hermetisch abgeschlossen sind, und zwar mit Hilfe von Schlauchleitung, die über Klemmvorrichtungen verfügt, die von Hand geöffnet und geschlossen werden können, um eine Übertragung der Inhaltsstoffe von einem Behälter zum anderen zu ermöglichen und dadurch alle Inhaltsstoffe der Behälter gegenüber der äußeren Umgebung zu isolieren.

5. Verfahren nach einem der Ansprüche 1 bis 3, **dadurch gekennzeichnet, dass** die Leukozyten mindestens einem sterilen Reagens unterworfen werden und die Leukozyten in einem sterilen Kulturmedium kultiviert werden.

6. Verfahren nach Anspruch 5, **gekennzeichnet durch** die Schritte des Einbringens der Leukozyten, des mindestens einen Reagens und des Kulturmediums in 3 separate sterile Behälter; Verbinden der Behälter untereinander und hermetisches Abschließen ihrer Inhaltsstoffe gegenüber der Atmosphäre **durch** sterile Schlauchleitung, die Vorrichtungen zur Regelung der Flüssigkeitsströmung aufweist und die geöffnet und geschlossen werden können; und Öffnen und Schließen der Vorrichtungen zur Regelung der Flüssigkeitsströmung nach Erfordernis, um die Inhaltsstoffe von einem Behälter zum anderen zu übertragen, während die Behälterinhaltsstoffe gegenüber der äußeren Umgebung isoliert sind.

7. Verfahren nach einem der Ansprüche 1 bis 6, **dadurch gekennzeichnet, dass** die Leukozytenfraktion einem osmotischen Schock unterworfen wird, indem die Leukozytenfraktion mit einem Volumen von destilliertem Wasser für eine Dauer zwischen 30 und 90 Sekunden gemischt wird, wobei das Volumen des destillierten Wassers das 15-fache von dem der Leukozytenfraktion beträgt.

8. Verfahren nach Anspruch 7, **dadurch gekennzeichnet, dass** die Isotonie der Leukozytenfraktion wiederhergestellt wird, indem eine hypertonische Lösung von einem Zehntel des Volumens des destillierten Wassers und die 10-fache Konzentration der isotonischen Lösung zugesetzt wird.

9. Verfahren nach Anspruch 8, **dadurch gekennzeichnet, dass** die hypertonische Lösung eine 9%ige Lösung von NaCl umfasst.

## Revendications

1. Procédé de culture de macrophages, **caractérisé par** les étapes consistant à séparer d'une quantité de sang initiale une fraction de globules blancs qui comprend un mélange de globules blancs et de globules rouges ayant une concentration de globules blancs plus forte que dans la quantité de sang initiale ; à soumettre ladite fraction de globules blancs à un choc osmotique qui détruit plus de globules rouges que de globules blancs ; à rétablir l'isotonie dans ladite fraction de globules blancs ; à additionner ladite fraction de globules blancs à un milieu de culture dans un récipient pour obtenir une suspension des globules blancs ; et à laisser incuber ledit milieu de culture.

2. Procédé selon la revendication 1, **caractérisé en ce que** le sérum préparé à partir de la quantité de sang initiale est additionné à ladite fraction de globules blancs après que l'isotonie ait été rétablie et avant que la fraction de globules blancs ait été mise à incuber dans un milieu de culture.

3. Procédé selon la revendication 2, **caractérisé en ce que** ledit sérum est préparé en séparant également de la quantité de sang initiale une fraction de plasma, en additionnant un agent coagulant à ladite fraction de plasma pour produire ledit sérum, et en séparant ledit sérum du plasma coagulé.

4. Procédé selon l'une quelconque des revendications 1-3, **caractérisé en ce que** la quantité de sang initiale, chaque fraction qui en est séparée, et chaque réactif utilisé dans le procédé, sont contenus dans des récipients séparés, lesquels récipients sont tous reliés les uns aux autres et hermétiquement isolés de l'atmosphère par des tubes ayant des dispositifs de serrage pouvant être ouverts ou fermés manuellement afin de permettre le transfert des contenus d'un récipient vers un autre pour de ce fait isoler tous les contenus des récipients de l'environnement extérieur.

5. Procédé selon l'une quelconque des revendications 1-3, **caractérisé par** l'exposition desdits globules blancs à au moins un réactif stérile et par la mise en culture desdits globules blancs dans un milieu de culture stérile.

6. Procédé selon la revendication 5, **caractérisé par** les étapes consistant à placer lesdits globules blancs, au moins ledit réactif, et ledit milieu de culture dans trois récipients stériles séparés ; à relier les uns aux autres lesdits récipients et à isoler hermétiquement leurs contenus de l'atmosphère par des tubes stériles possédant des régulateurs de débit de fluides pouvant être ouverts ou fermés ; et à ouvrir et fermer lesdits régulateurs de débit de fluides selon les instructions afin de transférer les contenus d'un récipient vers un autre, tout en maintenant les contenus des récipients isolés de l'environnement extérieur.

7. Procédé selon l'une quelconque des revendications 1-6, **caractérisé par** l'exposition de ladite fraction de globules blancs au choc osmotique en mélangeant la fraction de globules blancs avec un volume d'eau distillée pendant une période de temps comprise entre 30 et 90 secondes, ledit mélange comprenant un volume d'eau distillée 15 fois supérieur à celui de la fraction de globules blancs.

8. Procédé selon la revendication 7, **caractérisé en ce que** l'isotonie de la fraction de globules blancs est rétablie en ajoutant à la fraction de globules blancs une solution hypertonique contenant un dixième du volume de l'eau distillée et ayant dix fois la concentration d'une solution isotonique.

9. Procédé selon la revendication 8, **caractérisé en ce que** la solution hypertonique comprend une solution de NaCl à 9 %.
